# FASCICULE DE BREVET EUROPEEN

(11) **EP 0 890 347 B1**
(45) Date de publication et mention de la délivrance du brevet: **01.10.2003**
(21) Numéro de dépôt: 97440060.8
(22) Date de dépôt: 10.07.1997
(51) Int. Cl.: A61F 2/38

(54) **Prothèse de l'articulation du genou**
Kniegelenkprothese
Knee joint prosthesis

(43) Date de publication de la demande: 13.01.1999
(73) Titulaire: Société Ortho-Id, 69006 Lyon (FR); Noyer, Daniel, 38300 Maubec (FR); ROUSSOULY, Pierre, 69450 Saint Cyr au Mont d'Or (FR); Augoyard, Marc, 69160 Tassin la Demi Lune (FR)
(72) Inventeur: Noyer, Daniel, 38300 Maubec (FR); Roussouly, Pierre, 69450 Saint Cyr Au Mont D'Or (FR); Augoyard, Marc, 69160 Tassin La Demi Lune (FR); Roy, Christophe, 69006 Lyon (FR)
(74) Mandataire: Arbousse-Bastide, Jean-Claude Philippe

(56) Documents cités:
- DE-A- 3 314 038
- US-A- 4 808 185
- US-A- 5 219 362

## Description

La présente invention a pour objet une prothèse de l'articulation du genou.

L'articulation du genou permet les mouvements de flexion et d'extension de la jambe sur la cuisse, elle est réalisée entre le fémur et le tibia, par l'intermédiaire d'une part des extrémités de ceux-ci à savoir respectivement les condyles fémoraux et le plateau tibial, et d'autre part de la rotule et de la trochlée fémorale, et de ligaments qui empêchent un éventuel déboîtement.

Lors de la flexion, les condyles, qui sont de forme convexe, roulent et glissent sur le plateau tibial en permettant une importante flexion toutefois limitée par les masses molles.

De plus, la flexion s'accompagne d'une rotation axiale du tibia par rapport au fémur.

En hyperextension, le fémur et le tibia sont bloqués dans le prolongement l'un de l'autre, ce qui permet de garder la posture sans fatigue puisqu'aucun effort musculaire n'est nécessaire.

Lors d'une lésion articulaire du genou il est parfois nécessaire de recourir à la pose d'une articulation prothétique partielle ou totale du genou.

Les premières prothèses de l'articulation du genou étaient de simples charnières, ne permettant qu'un mouvement de flexion-extension par pivotement autour d'un axe transversal, ce qui présentait comme inconvénients d'une part une marche non naturelle du fait de la non reproduction du pivotement axial du tibia par rapport au fémur, et d'autre part l'impossibilité d'une flexion complète.

Les prothèses de genou connues à ce jour comportent pour la plupart un implant fémoral comprenant deux condyles et un bouclier trochléen, ce dernier étant destiné à coopérer avec une rotule prothétique ou non, tandis que les deux condyles s'articulent avec un implant tibial comportant un plateau recouvert d'un élément méniscal, mobile ou non, généralement réalisé en polyéthylène.

Ces prothèses sont dites à glissement, elles reproduisent le mouvement de l'articulation naturelle en combinant le roulement et le glissement des condyles sur l'élément méniscal.

Ces prothèses présentent toutes le même inconvénient à savoir une durée de vie moyenne relativement courte, de l'ordre d'un dizaine d'années, du fait de l'usure de l'élément méniscal.

En effet, la plupart des prothèses existantes comportent des condyles de forme convexe et de rayon non constant, et un élément méniscal présentant deux cavités concaves également de rayon non constant, le contact entre les condyles et l'élément méniscal est alors réalisé linéairement, en sorte que lors du mouvement de glissement il se produit à la longue un phénomène de laminage de la surface desdites cavités.

Afin d'augmenter la durée de vie des prothèses, certains ont proposé de diviser le phénomène d'usure en créant une deuxième surface de contact, par un possible glissement de l'élément méniscal sur le plateau tibial.

Puis d'autres ont proposé de dissocier le double mouvement de glissement et de roulement, le glissement étant réalisé entre l'élément méniscal et le plateau tibial, et le roulement entre ledit élément méniscal et les condyles, ces derniers étant de forme au moins partiellement sphérique permettant ainsi d'obtenir une congruence partielle ou totale.

Toutefois, ces prothèses présentent encore des inconvénients, notamment en ce qu'elles sont de conception complexe, en effet, elles mettent en oeuvre un grand nombre de pièces, ce qui multiplie les risques de dysfonctionnement, et nécessite un parfait ajustement qui allonge le temps opératoire au détriment du patient.

Dans le cas d'une hyperlaxité ligamentaire ou d'absence des ligaments croisés, notamment le ligament croisé postérieur, il est nécessaire de poser une prothèse dite stabilisée, dont la complexité est accrue par rapport aux autres prothèses.

Une prothèse stabilisée comporte généralement, en plus des caractéristiques précédemment décrites, soit un plot de centrage, muni ou non d'une tête sphérique, faisant saillie centralement du plateau tibial et coopérant avec l'implant fémoral en passant dans l'échancrure intercondylienne, soit une pièce en forme de crochet contre laquelle vient buter, lors de la flexion, une barrette transversale s'étendant dans Féchancrure intercondylienne et joignant les parties postérieures des condyles, soit un plot disposé dans la région centrale antérieure du plateau tibial sur laquelle vient buter la base du bouclier trochléen en position d'hyperextension.

Outre la complexité de la prothèse et de sa mise en place, il subsiste des inconvénients de limitation de la flexion; et surtout des risques de descellement de l'implant fémoral et/ou tibial suite au choc répétée lorsque les différents éléments mobiles viennent en butée contre un élément fixe.

Le document US A 52 193 62 décrit une prothèse de l'articulation du genou comprenant un implant fémoral à deux condyles interne et externe et un plateau tibial présentant deux cavités interne et externe coopérant chacune avec l'un desdits condyles. Toutefois, cette prothèse ne permet pas d'empêcher des effets de glissements en flexion, notamment en l'absence des ligaments croisés postérieur et antérieur. En outre, en flexion et en présence de ligaments croisés, le ligament croisé postéro-interne est détendu créant une situation d'instabilité.

Le document DE 33 140 38 décrit une prothèse de l'articulation du genou comprenant un implant fémoral à deux condyles, interne et externe, de forme convexe de rayon non constant et un implant tibial présentant deux cavités interne et externe d'appui des plans de contact respectivement des condyles interne et externe, le plan de contact du condyle interne étant entièrement de forme sphérique en sorte qu'en position de flexion ou d'extension ladite partie sphérique supporte tout le poids du patient ce qui fragilise à la longue la prothèse.

La présente invention a pour but de remédier à ces divers inconvénients en proposant une prothèse de l'articulation du genou, de conception simple, reproduisant le mouvement de l'articulation naturelle, d'une durée de vie supérieure à celle des prothèses existantes, utilisable en absence partielle ou totale des ligaments croisés, dont la mise en place est plus aisée, et qui assure un fonctionnement proche du mouvement physiologique du genou.

La prothèse de l'articulation du genou objet de la présente invention comprend d'une part un implant fémoral comportant un condyle interne et un condyle externe de forme convexe de rayon non constant et d'autre part un implant tibial présentant deux cavités interne et externe d'appui des plans de contact respectivement des condyles interne et externe, coopérant chacune avec l'un desdits condyles caractérisée en ce que ledit condyle interne comprend une partie médiane et une proéminence sphérique débordant du plan de contact dudit condyle interne dans sa région postérieure tandis que ledit implant tibial comporte, creusée dans la région postérieure de la cavité interne, une calotte sphérique destinés à recevoir de manière congruente une partie de ladite proéminence sphérique en sorte qu'en hyperextension les condyles reposent sur le plateau tibial, le condyle interne reposant à la fois par sa partie médiane et par sa partie postérieure sphérique engagée dans ladite calotte sphérique.

En fin d'extension, dans la posture debout, et en début de flexion, il se produit, comme pour l'articulation naturelle, un transfert de charge sur le condyle interne, en sorte que l'effort est principalement supporté par les parties sphériques congruentes, où il est de ce fait régulièrement réparti limitant ainsi le phénomène d'usure.

Lors du passage en flexion, le contact entre le condyle interne et le plateau tibial est réalisé principalement par les parties sphériques, en sorte qu'il ne se produit pas de roulement mais uniquement un glissement ce qui limite l'usure.

D'autre part les parties sphériques permettent, outre la flexion, un pivotement du tibia dans le sens axial, tandis que le condyle externe roule et glisse sur ledit plateau, ce qui permet reproduire le mouvement naturel.

On notera que lors du mouvement flexion, que ce soit lors du passage de l'extension à la flexion, ou inversement, l'effort est principalement supporté par le condyle interne, en sorte que lors du double mouvement de glissement et de roulement du condyle externe, l'usure de la matière est minimisée.

D'autre part, la flexion peut être réalisée totalement, uniquement limitée par les masses molles.

Dans le cas d'absence de ligaments croisés, que ce soit en flexion ou en extension, la prothèse est stabilisée du fait de la congruence des parties sphériques qui interdit les phénomènes de tiroir.

Selon une caractéristique additionnelle de la prothèse selon l'invention, la proéminence sphérique est échancrée intérieurement au niveau de l'échancrure intercondylienne, dans le prolongement de la gorge de passage de la rotule sur le bouclier trochléen.

Selon une autre caractéristique additionnelle de la prothèse selon l'invention, la face d'accouplement de l'implant tibial au tibia comporte une partie plane de laquelle font saillie deux calottes sphériques identiques.

La pose de l'implant tibial ne nécessite, outre le perçage d'un trou pour la mise en place d'une quille, qu'une résection plane et la réalisation de deux cavités sphériques au moyen d'une fraise sphérique, ce qui simplifie l'intervention du chirurgien.

Les avantages et les caractéristiques du véhicule selon l'invention, ressortiront plus clairement de la description qui suit et qui se rapporte au dessin annexé, lequel en représente des modes de réalisation non limitatifs.

Dans le dessin annexé:
- la figure 1 représente une vue de profil et en éclaté d'une prothèse selon l'invention.
- la figure 2 représente une vue postérieure de la même prothèse.
- la figure 3 représente une vue de dessus de l'implant tibial de la même prothèse.

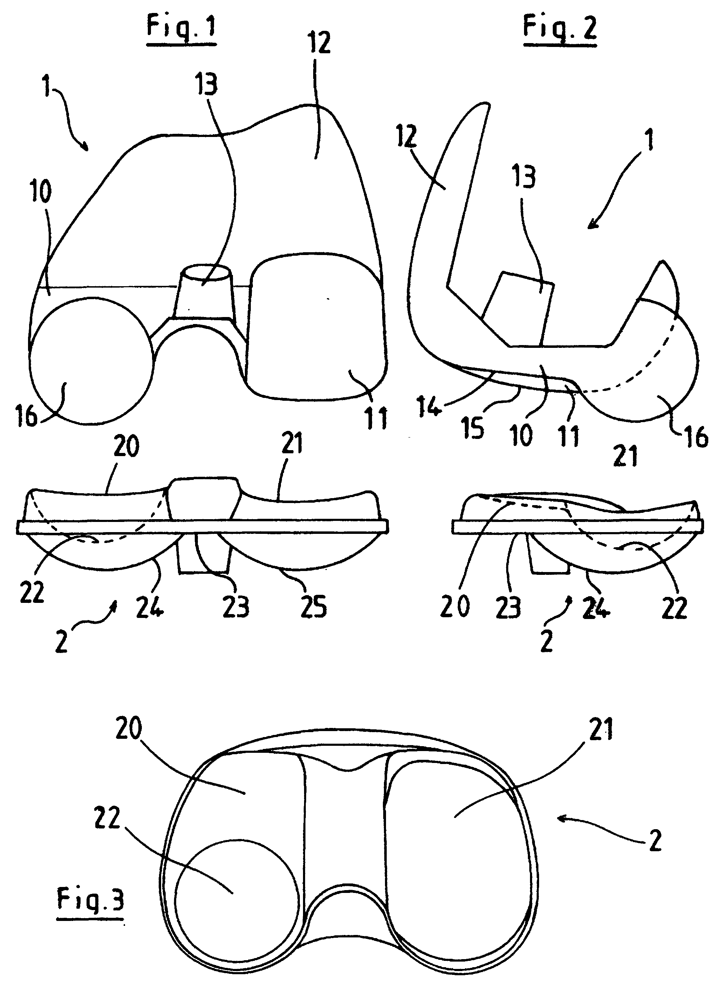

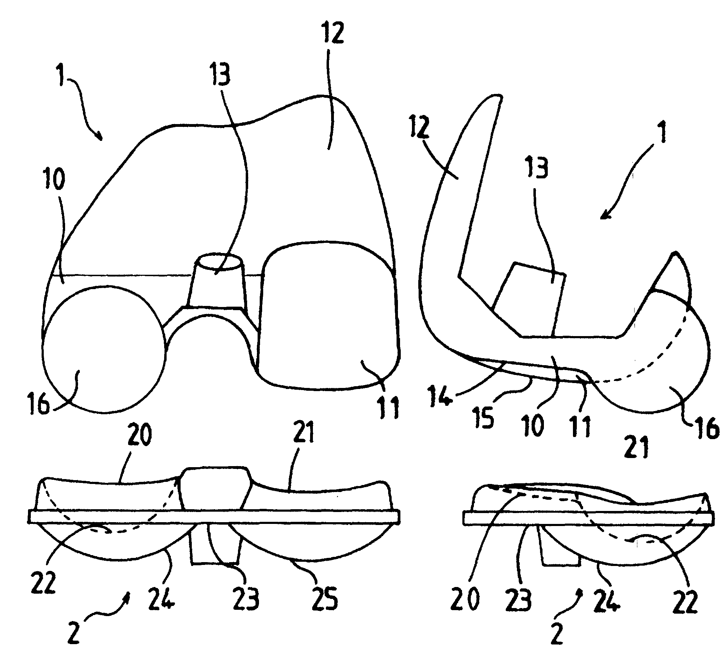

Si on se réfère aux figures 1 et 2, on peut voir qu'une prothèse selon la présente invention comprend un implant fémoral 1 et un implant tibial 2.

L'implant fémoral 1 comporte deux condyles, un condyle interne 10 et un condyle externe 11, un bouclier trochléen 12 et un plot 13 de solidarisation d'un moyen de fixation du type quille, non représentée, tandis que l'implant tibial, que l'on peut également voir sur la figure 3, comporte deux cavités 20 et 21 d'appui des faces de contact respectivement 14 et 15 des condyles respectivement 10 et 11.

Le condyle interne 10 comporte dans sa région postérieure une proéminence sphérique 16 débordant la face d'appui 14, tandis que l'implant tibial comporte, creusée dans la région postérieure de la cavité 20, une calotte sphérique 22 destinée à recevoir de manière congruente une partie de la proéminence sphérique 16.

Afin de garantir une épaisseur sensiblement constante du plateau tibial 2 au droit de la calotte sphérique 22 sans nécessiter d'accroître l'épaisseur même du plateau tibial 2, la face inférieure 23 de celui-ci, qui est de forme générale plane, est bombée et forme une calotte sphérique convexe 24. Cette calotte sphérique 24 est reproduite à l'identique en une calotte sphérique convexe 25 au droit de la région postérieure de la cavité 21.

Les calottes sphériques 24 et 25 permettent, par rapport aux prothèses classiques dont l'implant tibial comporte une face inférieure plate, une meilleure répartition des charges lors de la marche.

D'autre part, les calottes sphériques 24 et 25 permettent d'éviter un phénomène de cisaillement en sorte que cela autorise un scellement de l'implant tibial avec une quille de longueur réduite, voire sans quille.

Lors de la pose de l'implant tibial 2, après une résection plane de la matière osseuse, on pratique, au moyen d'un gabarit et d'une fraise sphérique, deux cavités en forme de calotte sphérique, destinées à loger les calottes sphériques 24 et 25, ce qui simplifie l'intervention.

## Revendications

1. Prothèse de l'articulation du genou comprenant d'une part un implant fémoral (1) comportent un condyle interne (10) et un condyle externe (il) de forme convexe de rayon non constant et d'autre part un implant tibial (2) présentant deux cavités interne (20) et externe (21) d'appui des plans de contact (14, 15) respectivement des condyles interne (10) et externe (11), coopérant chacune avec l'un desdits condyles (10, 11) **caractérisée en ce que** ledit condyle interne (11) comprend une partie médiane et une proéminence sphérique (16) débordant du plan de contact (14) dudit condyle interne (10) dans sa région postérieure tandis que ledit implant tibial (2) comporte, creusée dans la région postérieure de la cavité interne (20), une calotte sphérique (22) destinée à recevoir de manière congruente une partie de ladite proéminence sphérique (16) en sorte qu'en hyperextension le condyle interne (10) repose à la fois par sa partie médiane et par sa partie postérieure sphérique engagée dans ladite calotte sphérique (22).

2. Prothèse selon la revendication 1 **caractérisée en ce que** la proéminence sphérique (16) est échancrée intérieurement au droit de l'échancrure intercondylienne, dans le prolongement de la gorge dè passage de la rotule sur le bouclier trochléen (12).

3. Prothèse selon la revendication 1 ou la revendication 2 **caractérisée en ce que** la face d'accouplement de l'implant tibial au tibia comporte une partie plane (23) de laquelle font saillie deux calottes sphériques (24, 25) identiques destinées à être implantées dans deux cavités sphériques pratiquées dans la matière osseuse au moyen d'une fraise sphérique.

## Patentansprüche

1. Prothese für das Kniegelenk, bestehend einerseits aus einem Oberschenkelimplantat (1) mit einem internen Gelenkkopf (10) und einem externen Gelenkkopf (11), der eine konvexe Formt mit nicht konstantem Radius aufweist, und andererseits aus einem Schienbeinimplantat (2) mit einer internen Vertiefung (20) und einer externen Vertiefung (21) zum Stützen der Kontaktflächen (14, 15) des internen bzw. externen Gelenkkopfes (10, 11), wobei jede Vertiefung mit einem der Gelenkköpfe (10, 11) zusammenwirkt, **dadurch gekennzeichnet, daß** der interne Gelenkkopf (10) einen mittleren Teil und einen sphärischen Vorsprung (16) aufweist, der über die Kontaktfläche (14) des internen Gelenkkopfes (10) in dessen hinteren Bereich hinausragt, während der Schienbeinimplantat (2) eine im hinteren Bereich der internen Vertiefung (20) ausgehöhlte sphärische Kappe (22) aufweist, die dazu vorgesehen ist, einen Teil des sphärischen Vorsprungs (16) kongruent aufzunehmen, so daß bei Hyperextension der interne Gelenkkopf (10) sowohl mit seinem mittleren Teil als auch mit seinem hinteren sphärischen Teil in der sphärischen Kappe (22) aufliegt.

2. Prothese nach Anspruch 1, **dadurch gekennzeichnet, daß** der sphärische Vorsprung (16) auf der Innenseite senkrecht auf die Aussparung zwischen den Gelenkköpfen in Verlängerung der Durchgangsnut von der Kniescheibe bis zum trochleären Schild (12) ausgespart ist.

3. Prothese nach Anspruch 1 oder 2, **dadurch gekennzeichnet, daß** die Fläche zum Ankoppeln des Schieneinimplantats an das Schienbein einen ebenen Teil (24, 25) aufweist, aus welchem zwei identische sphärische Kappen (24, 25) hervorstehen, die dazu vorgesehen sind, in zwei sphärische Vertiefungen implantiert zu werden, die im Knochenmaterial mit einem Kugelfräser ausgeführt sind.

## Claims

1. Prosthesis of the knee joint including on the one hand a femoral implant (1) consisting of an internal condyle (10) and an external condyle (11) convex in shape with a non-constant radius, and on the other hand of a tibial implant (2) presenting two cavities, internal (20) and external (21), respectively supporting the contact faces (14, 15) of the internal (10) and external (11) condyles, each cooperating with one of the said condyles (10,11) **characterised in that** the said internal condyle (11) includes a centre section and a spherical prominence (16) projecting beyond the contact face (14) of the rear part of the said internal condyle (10), whereas the said tibial implant (2) includes, hollowed out in the rear part of the internal cavity (20) a spherical cap (22) intended to receive congruently a part of the said spherical prominence (16) so that in hyperextension the internal condyle (10) rests at once by its centre section and its spherical rear part engaged in the said spherical cap (22).

2. Prosthesis according to claim 1 **characterised in that** the spherical prominence (16) is notched on the inside at the level of the intercondylar notch, in continuation of the femoral trochlear groove on the trochlear shield (12).

3. Prosthesis according to claim 1 or claim 2 **characterised in that** the coupling face of the tibial implant onto the tibia includes a flat part (23) from which project two identical spherical caps (24, 25) intended to be implanted in two spherical cavities made in the bony matter using a spherical bone drill.
